# FASCICULE DE BREVET EUROPEEN

(11) **EP 0 638 543 B1**
(45) Date de publication et mention de la délivrance du brevet: **11.11.1998**
(21) Numéro de dépôt: 94401829.0
(22) Date de dépôt: 09.08.1994
(51) Int. Cl.: C07C 69/743, A01N 53/00, C07D 209/48, C07D 307/42, C07D 277/24

(54) **Nouveaux esters dérivés de l'acide 2,2-diméthyl 3-(3,3,3-trifluoro-1-propényl) cyclopropane carboxylique, leur procédé de préparation et leur application comme pesticides**
2,2-Dimethyl-3-(3,3,3-Trifluor-1-Propenyl) Cyclopropancarbonsäureesterderivate, ihre Herstellung und ihre Verwendung als Pestizide
Esters of 2,2-dimethyl 3-(3,3,3-trifluoro-1-propenyl) cyclopropanecarboxylic acid, their preparation and use as pesticides

(30) Priorité: 10.08.1993 FR 9309804
(43) Date de publication de la demande: 15.02.1995
(73) Titulaire: ROUSSEL UCLAF, 93230 Romainville (FR)
(72) Inventeur: Babin, Didier, F-78180 Montigny (FR); Benoit, Marc, F-13360 Roquevaire (FR); Demoute, Jean-Pierre, F-93360 Neuilly Plaisance (FR)
(74) Mandataire: Vieillefosse, Jean-Claude

(56) Documents cités:
- EP-A- 0 003 336
- EP-A- 0 031 199
- EP-A- 0 194 919
- EP-A- 0 551 035
- EP-A- 0 556 123
- EP-A- 0 557 192
- FR-A- 2 392 964

## Description

La présente invention concerne de nouveaux esters dérivés de l'acide 2,2-diméthyl 3-[3,3,3-trifluoro 1-propényl] cyclopropane carboxylique, leur procédé de préparation et leur application comme pesticides. On connaissait déjà des dérivés d'acide perhaloalkylvinylcyclopropane carboxylique doués de propriétés insecticides (cf EP 3336).

L'invention a pour objet sous toutes les formes stéréoisomères possibles ainsi que leurs mélanges, les composés de formule (I) : 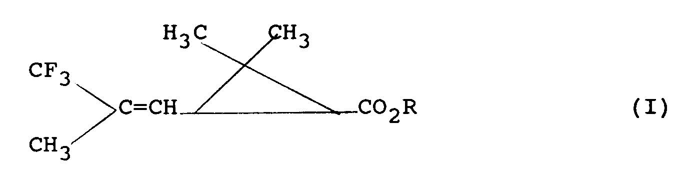 dans laquelle R représente un radical choisi dans le groupe constitué par les radicaux :

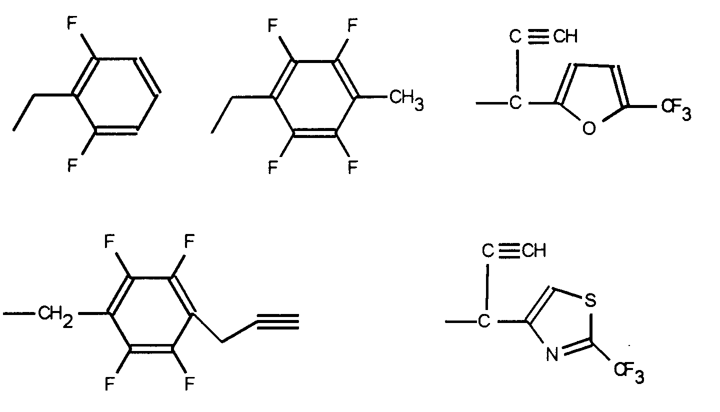

L'invention a particulièrement pour objet les composés de formule (I) dans lesquels la copule cyclopropanique est de structure 1Rcis, ainsi que ceux dans laquelle la géométrie de la double liaison est E.

L'invention a plus particulièrement pour objet les composés de formule (I) dont la préparation est donnée ci-après dans la partie expérimentale et tout spécialement les composés de formule (I) dans lesquels R représente le radical [(4-méthyl 2,3,5,6-tétrafluoro) phényl] méthyle, ceux dans lesquels R représente un radical 4-(2-propyn-1-yl) 2,3,5,6-tétrafluoro) phényl] méthyle, ainsi que ceux dans lesquels R représente un radical 1-[5-trifluorométhyl-2-furyl] 2 propynyle ou encore dans lesquels R représente un radical cyano(3-phénoxy 4-fluorophényl) méthyle.

L'invention a également pour objet un procédé de préparation des composés de formule (I) caractérisé en ce que l'on soumet un acide de formule (II) : 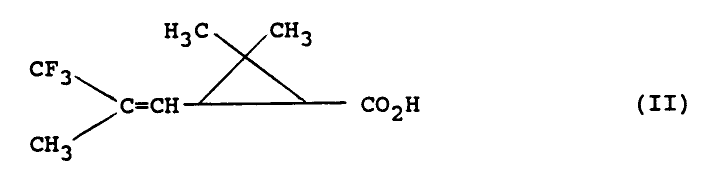 ou un dérivé fonctionnel de cet acide à l'action d'un alcool de formule (III) :

ROH (III)

dans laquelle R conserve sa signification précédente ou d'un dérivé fonctionnel de cet alcool, pour obtenir le composé de formule (I) correspondant.

Le dérivé fonctionnel d'acide utilisé est de préférence un chlorure d'acide. Lorsque l'on fait réagir l'acide de formule (II) et l'alcool de formule (III), on opère de préférence en présence de dicyclohexylcarbodiimide.

La préparation de l'acide [1R(1α,3α)] 2,2-diméthyl 3-[(E)-2-méthyl 3,3,3-trifluoro 1-propényl] cyclopropane carboxylique est donnée ci-après dans la partie expérimentale.

Les différents alcools utilisés sont des produits connus; ainsi l'α-éthynyl 2-(trifluorométhyl) 4-thiazole méhtanol peut être préparé comme indiqué dans la demande de brevet européen n° 0 556 123.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment au moins l'un des produits de formule (I) définis ci-dessus.

L'invention a notamment pour objet les compositions insecticides renfermant comme principe actif au moins l'un des produits définis ci-dessus.

L'invention a plus particulièrement pour objet les compositions insecticides définies ci-dessus, destinées à la lutte contre Diabrotica et les autres parasites du sol.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisi dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention.

### Exemple 1: [1R-[1α,3α(E)]-2,2-diméthyl-3-(2-méthyl-3,3,3-trifluoro-1-propen-1-yl) cyclopropane carboxylate de [(4-méthyl-2,3,5,6-tétrafluorophényl)] méthyle.

On introduit à 0°C une solution renfermant 650 mg de dicyclohexylcarbodiimide, 10 mg de 4-diméthylaminopyridine et 3,3 ml de chlorure de méthylène dans une solution renfermant 700 mg d'acide [1R-[1α,3α(E)]-2,2-diméthyl-3-(2-méthyl-3,3,3-trifluoro-1-propen-1-yl) cyclopropane carboxylique, 673 mg d'alcool 4-méthyl 2,3,5,6-tétrafluorobenzylique et 10 ml de chlorure de méthylène.

On laisse la température revenir à 20°C, on agite pendant 16 heures et filtre. On rince au chlorure de méthylène et amène à sec sous pression réduite. On obtient 1,22 g de produit que l'on chromatographie sur silice en éluant avec le mélange hexane-éther isopropylique 95-5. On isole 1,22 g de produit recherché rf=0,2.
**RMN CDCl**_{**3**} **ppm**
   - 1,26(s) 1,28(s): H des CH₃ géminés
   - 1,84 d,J=1: H du CH₃ porté par la double liaison
   - 1,80 à 1,95 m: H₁ et H₃
   - 2,29(t,J=α): H du 4 méthyl
   - 5,19: H du CH₂ en α du C=O

En opérant comme à l'exemple 1 à partir des alcools correspondants on a préparé les produits suivants.

### Exemple 2 : [1R-(1α,3α(E)] 2,2-diméthyl-3-[2-méthyl-3,3,3-trifluoro-1-propen-1-yl] cyclopropane carboxylate de (2,6-difluorophényl) méthyle.

**RMN CDCl**_{**3**} **ppm**
   - 1,24 et 1,28: H des CH₃ géminés
   - 1,86 m: H₁ et H₃
   - 1,84: H du méthyle vinylique
   - 5,19: H du CH₂ en α du C=O
   - 6,39: H vinylique
   - 6,92: H en 3 et 5 du phényle
   - 7,32: H en 4 du phényle

### Exemple 3 : [1R-(1α,3α(E))]-2,2-diméthyl-3(2-méthyl-3,3,3-trifluoro propen-1-yl) cyclopropane carboxylate de [5-trifluorométhyl-2-furyl) 2-propynyle.

**RMN CDCl**_{**3**} **ppm**
   - 1,26 ; 1,27 ; 1,28 ; 1,30: H des méthyles géminés
   - 1,82 et 1,85: H du CH₃ vinylique
   - 2,64: H de l'éthynyle
   - 6,38: H vinylique
   - 6,46 et 6,50: H du CH en α de la triple liaison

### Exemple 4 : [1R-(1α,3α(E)]-2,2-diméthyl-3(2-méthyl 3,3,3-trifluoro-1-propen 1-yl) cyclopropane carboxylate de [(4-(2-propyn-1-yl)-2,3,5,6-tétrafluoro) phényl] méthyle.

**RMN CDCl**_{**3**}
   - 1,26 et 1,28: H des CH₃ géminés
   - 1,83: H du méthyle vinylique
   - 1,86: H₁ et H₃
   - 6,37: H éthylénique
   - 3,64: H du CH₂ en α de la triple liaison
   - 5,20: H

### Exemple 5 : [1R-[1α,3α(E)]-2,2-diméthyl-3-(2-méthyl-3,3,3-trifluoro 1-propen-1-yl) cyclopropane carboxylate de [2-trifluorométhyl-3-isothiazyl] 2-propynyle.

**RMN CDCl**_{**3**} **ppm**
   - 1,26(s) 1,27(s): H des méthyles géminés
   - 1,28(s) 1,31(s):
   - 1,82 d,J=1: CH₃-vinylique
   - 1,84 d,J=1:
   - 1,93 (m): H₁ et H₃
   - 2,68 d,J=2,5: H de la triple liaison
   - 2,70 d,J=2,5:

### PREPARATION 1 : Acide [1R(1alpha,3alpha)] 2,2-diméthyl 3-[(E)-2-méthyl 3,3,3-trifluoro 1-propényl] cyclopropane carboxylique.

### STADE A : 3-(2-oxopropyl) 2,2-diméthyl cyclopropane carboxylate de méthyle.

On chauffe à 30°/34°C 10 g d'acide 3-(2-oxopropyl) 2,2-diméthyl cyclopropane carboxylique dans 100 cm³ d'acétone en présence de 7,35 g de carbonate acide de potassium et 5 cm³ de diméthylsulfate. Après 4 heures d'agitation, on ajoute 0,85 cm³ de diméthylsulfate et maintient 20 heures à 30°/34°C. On filtre, reprend à l'éther éthylique, évapore le solvant, chromatographie le résidu sur silice (éluant : hexane-acétate d'éthyle 8-2) et obtient 10,43 g de produit attendu.

### STADE B : 3-(2-méthyl 2-triméthylsilyloxy 3,3,3-trifluoropropyl) 2,2-diméthyl cyclopropane carboxylate de méthyle.

A 5 g de produit obtenu au stade A et 8 cm³ de trifluorométhyl triméthylsilane dans 65 cm³ de tétrahydrofuranne, on ajoute à 0°C, 0,5 cm³ de fluorure de tétrabutylammonium et agite 15 minutes. On verse dans une solution aqueuse glacée de phosphate acide de potassium, extrait à l'éther éthylique, sèche et évapore le solvant. On obtient 7,57 g de produit attendu.

### STADE C : 3-(2-méthyl 2-hydroxy 3,3,3-trifluoropropyl) 2,2-diméthyl cyclopropane carboxylate de méthyle.

On ajoute à température ambiante 444 mg de fluorure de potassium à 500 mg de produit obtenu au stade B dans 5 cm³ de méthanol. On agite 3 heures, verse dans 25 cm³ d'une solution aqueuse de phosphate acide de potassium, extrait à l'éther éthylique, sèche et évapore le solvant. On obtient 360 mg de produit attendu.

### STADE D : 2,2-diméthyl 3-[(E) 2-méthyl 3,3,3-trifluoro 1-propényl] cyclopropane carboxylate de méthyle.

On ajoute à 0°C 160 ml de chlorure de thionyle à 80,5 g de 2,2-diméthyl 3-(2-méthyl 2-hydroxy 3,3,3-trifluoropropyl) cyclopropane carboxylate de méthyle obtenu au stade C. On chauffe à reflux pendant 48 h, refroidit, verse sur de l'eau glacée, extrait à l'éther isopropylique et lave les phases organiques à l'eau. Après séchage sur sulfate de magnésium et évaporation à sec, le brut est chromatographié sur silice avec le mélange hexane-AcOEt (9-1) puis rechromatographié avec le mélange hexane-acétone (99-1). On isole l'alcool de départ (22,02 g) le mélange des isomères exo + Z (11,71 g) et le produit attendu (23,31 g).

### STADE E : Acide [1R(1alpha,3alpha)] 2,2-diméthyl 3-[(E)-2-méthyl 3,3,3-trifluoro 1-propényl] cyclopropane carboxylique.

On chauffe à 60°C pendant 2 heures et demie 4,14 g de produit obtenu au stade D dans 52,6 cm³ de méthanol en présence de 19,3 cm³ de soude N puis 30 minutes en présence de 2 cm³ de soude N supplémentaires. On verse le milieu réactionnel dans l'eau glacée, extrait à l'éther isopropylique, acidifie la phase aqueuse avec KH₂PO₄, extrait celle-ci avec de l'éther isopropylique. Ces dernières phases sont séchées et évaporées sous pression réduit. On obtient 2,94 g de produit attendu.
- 1,84 (s): CH₃ de la double liaison
- 1,26 et 1,22 (s): méthyl géminés
- 1,3 à 1,9 (m): H₁ et H₃
- 6,43 (m): H vinylique.

### EXEMPLE 6 : Préparation d'un concentré soluble

On effectue un mélange homogène de :

| | |
|---|---|
| Produit de l'exemple 1 | 0,25 g |
| Butoxyde pipéronyle | 1,00 g |
| Tween 80 | 0,25 g |
| Topanol A | 0,1 g |
| Eau | 98,4 g |

### EXEMPLE 7 : Préparation d'un concentré émulsifiable

On mélange intimement :

| | |
|---|---|
| Produit de l'exemple 1 | 0,015 g |
| Butoxyde de pipéronyle | 0,5 g |
| Topanol A | 0,1 g |
| Tween 80 | 3,5 g |
| Xylène | 95,885 g |

### ETUDE BIOLOGIQUE

### A - Activité sur Diabrotica

Les insectes tests sont des larves de dernier stade de Diabrotica.

On traite une rondelle de papier filtre de 9 cm de diamètre, déposée au fond d'une boîte de Pétri, à l'aide de 2 cm³ de solution acétonique du produit à tester. Après séchage on dépose 15 larves par dose et on effectue le contrôle de mortalité 24 heures après le traitement.

Dès la dose de 1 ppm les produits de l'invention présentent une bonne activité.

### Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement, les individus sont placés sur un milieu nutritif artificiel (milieu de Poitout).

On effectue le contrôle des mortalités 48 heures après traitement.

Les produits de l'invention présentent des résultats intéressants.

## Revendications

1. Sous toutes les formes stéréoisomères possibles ainsi que leurs mélanges, les composés de formule (I) : 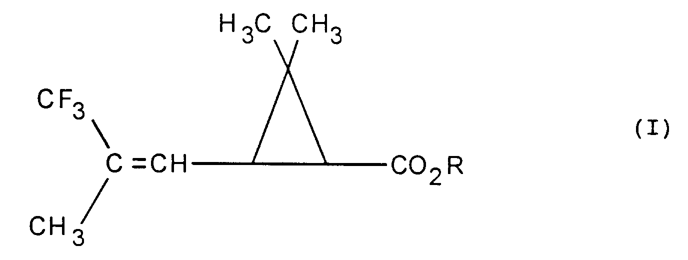 dans laquelle R représente un radical choisi dans le groupe constitué par les radicaux :

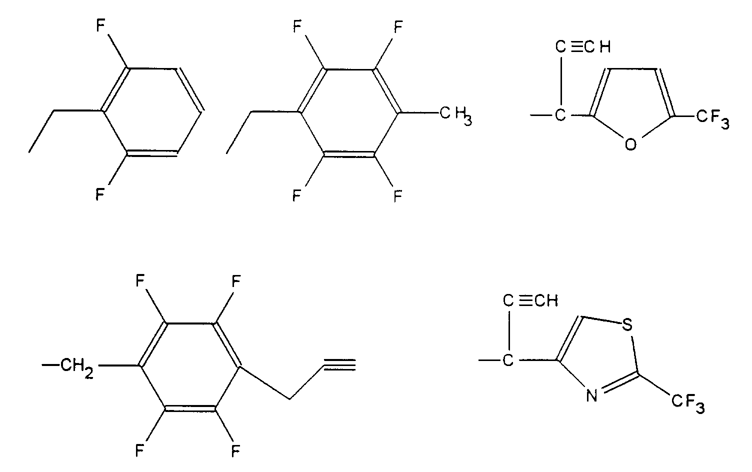

2. Les composés de formule (I) tels que définis à la revendication 1 dans lesquels la copule cyclopropanique est de structure 1Rcis.

3. Les composés de formule (I) tels que définis à la revendication 1 ou 2 dans lesquels la géométrie de la double liaison est E.

4. Les composés de formule (I) tels que définis à l'une quelconque des revendications 1 à 3 dans lesquels R représente le radical [(4-méthyl 2,3,5,6-tétrafluoro) phényl] méthyle.

5. Les composés de formule (I) tels que définis à l'une quelconque des revendications 1 à 3 dans lesquels R représente un radical 4-(2-propyn-1-yl) 2,3,5,6-tétrafluoro) phényl] méthyle.

6. Les composés de formule (I) tels que définis à l'une quelconque des revendications 1 à 3 dans lesquels R représente un radical 1-[5-trifluorométhyl-2-furyl] 2-propynyle.

7. Les composés de formule (I) tels que définis à l'une quelconque des revendications 1 à 5 dans lesquels R représente un radical cyano(3-phénoxy 4-fluorophényl) méthyle.

8. Procédé de préparation des composés de formule (I) tels que définis à l'une quelconque des revendications 1 à 7 caractérisé en ce que l'on soumet un acide de formule (II) : 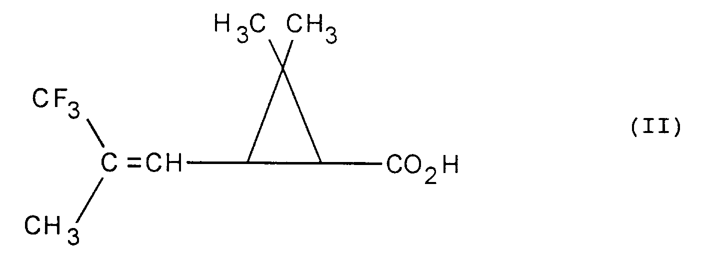 ou un dérivé fonctionnel de cet acide à l'action d'un alcool de formule (III) :
ROH (III)
dans laquelle R conserve sa signification précédente ou d'un dérivé fonctionnel de cet alcool, pour obtenir le composé de formule (L) correspondant.

9. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 7.

10. Les compositions insecticides, caractérisées en ce qu'elles renferment comme principe actif au moins un composé défini à l'une quelconque des revendications 1 à 7.

11. Les compositions insecticides définies à la revendication 10, caractérisées en ce qu'elles sont destinées à la lutte contre DIABROTICA et les autres parasites du sol.

12. Les compositions acaricides et nématicides, caractérisées en ce qu'elles renferment comme principe actif au moins un composé défini à l'une quelconque des revendications 1 à 7.

13. Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibiomovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

## Claims

1. In all the possible stereoisomeric forms as well as their mixtures, the compounds of formula (I): 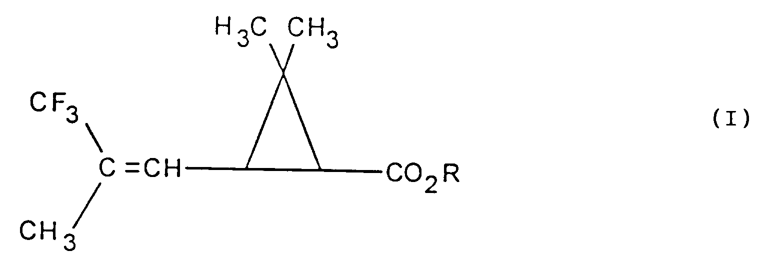 in which R represents a radical chosen from the group constituted by the radicals:

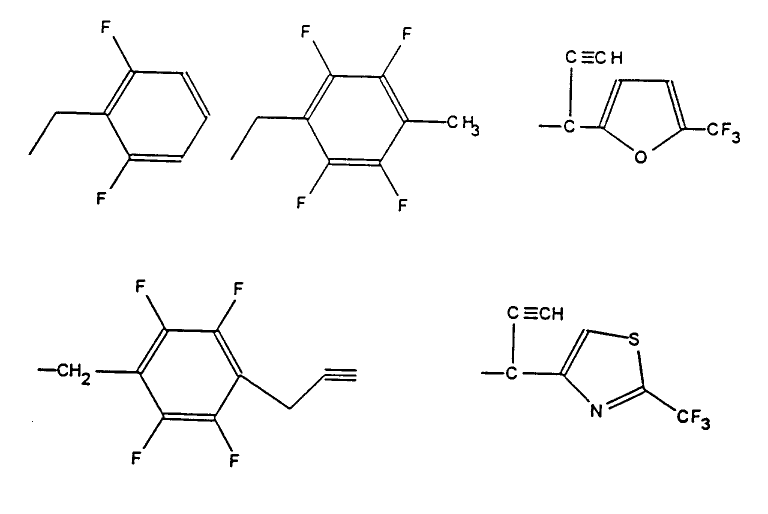

2. The compounds of formula (I) as defined in claim 1 in which the cyclopropane copula is of 1Rcis structure.

3. The compounds of formula (I) as defined in claim 1 or 2 in which the geometry of the double bond is E.

4. The compounds of formula (I) as defined in any one of claims 1 to 3 in which R represents the [(4-methyl 2,3,5,6-tetrafluoro) phenyl] methyl radical.

5. The compounds of formula (I) as defined in any one of claims 1 to 3 in which R represents a 4-(2-propyn-1-yl) 2,3,5,6-tetrafluoro) phenyl] methyl radical.

6. The compounds of formula (I) as defined in any one of claims 1 to 3 in which R represents a 1-[5-trifluoromethyl-2-furyl] 2-propynyl radical.

7. The compounds of formula (I) as defined in any one of claims 1 to 5 in which R represents a cyano(3-phenoxy 4-fluorophenyl) methyl radical.

8. Preparation process for the compounds of formula (I) as defined in any one of claims 1 to 7 characterized in that an acid of formula (II): 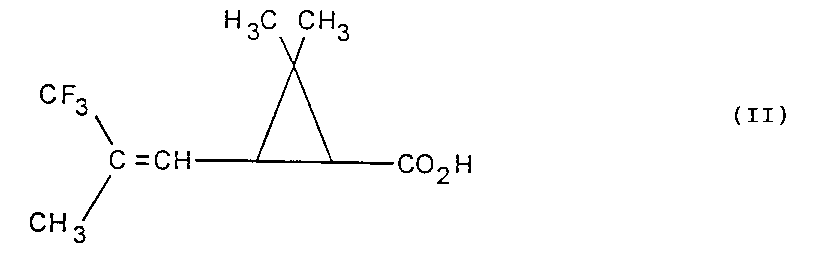 or a functional derivative of this acid, is subjected to the action of an alcohol of formula (III):
ROH (III)
in which R retains its previous meaning, or a functional derivative of this alcohol, in order to obtain the corresponding compound of formula (I).

9. The compositions intended for combating parasites of vegetation, parasites of premises and parasites of warm-blooded animals, characterized in that they contain as active ingredient at least one compound defined in any one of claims 1 to 7.

10. The insecticide compositions, characterized in that they contain as active ingredient at least one compound defined in any one of claims 1 to 7.

11. The insecticide compositions defined in claim 10, characterized in that they are intended for combating DIABROTICA and other parasites of the soil.

12. The acaricide and nematicide compositions, characterized in that they contain as active ingredient at least one compound defined in any one of claims 1 to 7.

13. Combinations endowed with insecticide, acaricide or nematicide activity, characterized in that they contain as active ingredient, on the one hand at least one of the compounds of general formula (I) and on the other hand, at least one of the pyrethrinoid esters chosen from the group constituted by the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohol with chrysanthemic acids, by the esters of 5-benzyl-3-furyl methyl alcohol with 2,2-dimethyl-3-(2-oxo-3-tetrahydrothiophenylidenemethyl)-cyclopropanecarboxylic acids, by the esters of 3-phenoxybenzyl alcohol and of alpha-cyano-3-phenoxybenzyl alcohol with 2,2-dimethyl-3-(2,2-dichlorovinyl)cyclopropanecarboxylic acids, by the esters of alpha-cyano-3-phenoxy-benzyl alcohol with 2,2-dimethyl-3-(2,2-dibromovinyl)-cyclopropanecarboxylic acids, by the esters of 3-phenoxybenzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol, and of alphacyano-3-phenoxybenzyl alcohol with 2,2-dimethyl-3-(1,2,2,2-tetrahaloethyl)-cyclopropanecarboxylic acids, in which "halo" represents a fluorine, chlorine or bromine atom, it being understood that the compounds (I) can exist in all their possible stereoisomeric forms, as well as the acid and alcohol copulas of the above pyrethrinoid esters.

## Patentansprüche

1. In sämtlichen ihrer möglichen stereoisomeren Formen sowie in Form von deren Gemischen die Verbindungen der Formel (I): 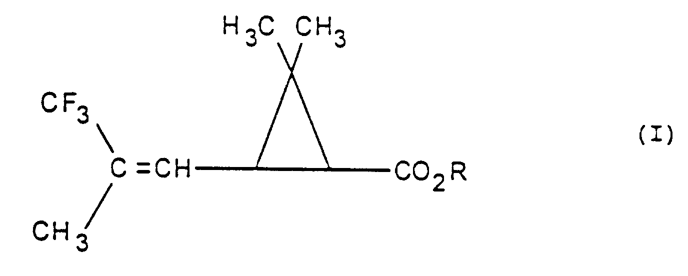 worin R für einen der folgenden Reste steht

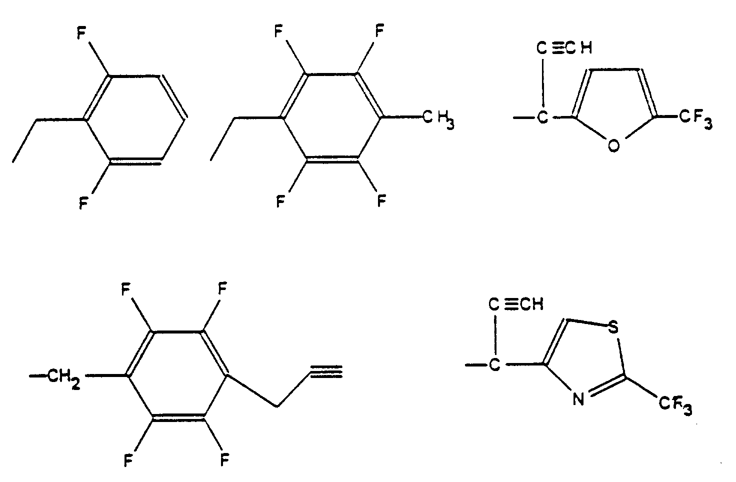

2. Verbindungen der Formel (I), wie in Anspruch 1 definiert, worin die Cyclopropanverknüpfung die 1R-cis-Struktur besitzt.

3. Verbindungen der Formel (I), wie in Anspruch 1 oder 2 definiert, worin die Geometrie der Doppelbindung die E-Geometrie ist.

4. Verbindungen der Formel (I), wie in einem der Ansprüche 1 bis 3 definiert, worin R den [(4-Methyl-2,3,5,6-tetrafluor)-phenyl]-methylrest bedeutet.

5. Verbindungen der Formel (I), wie in einem der Ansprüche 1 bis 3 definiert, worin R den[4-(2-Propin-1-yl)-2,3,5,6-tetrafluor -phenyl]-methylrest bedeutet.

6. Verbindungen der Formel (I), wie in einem der Ansprüche 1 bis 3 definiert, worin R den 1-(5-Trifluormethyl-2-furyl)-2-propinylrest bedeutet.

7. Verbindungen der Formel (I), wie in einem der Ansprüche 1 bis 5 definiert, worin R den Cyano-(3-phenoxy-4-fluorphenyl)-methylrest bedeutet.

8. Verfahren zur Herstellung der Verbindungen der Formel (I), wie in einem der Ansprüche 1 bis 7 definiert,dadurch gekennzeichnet, daß man eine Säure der Formel (II) 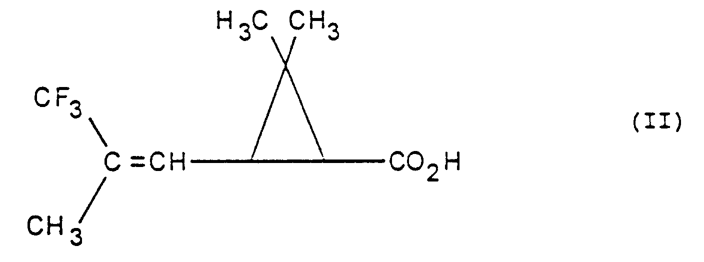 oder ein funktionelles Derivat dieser Säure der Einwirkung eines Alkohols der Formel (III)
ROH (III)
worin R die vorstehend angegebene Bedeutung besitzt, oder der Einwirkung eines funktionellen Derivats dieses Alkohols unterzieht, um zu der entsprechenden Verbindung der Formel (I) zu gelangen.

9. Zusammensetzungen für die Bekämpfung von Pflanzenparasiten, Parasiten von Räumlichkeiten und Parasiten warmblütiger Tiere, dadurch gekennzeichnet, daß sie als Wirkstoff zumindest eine Verbindung, wie in einem der Ansprüche 1 bis 7 definiert, enthalten.

10. Insektizide Zusammensetzungen, dadurch gekennzeichnet, daß sie als Wirkstoff zumindest eine Verbindung, wie in einem der Ansprüche 1 bis 7 definiert, enthalten.

11. Insektizide Zusammensetzungen, wie in Anspruch 10 definiert, dadurch gekennzeichnet, daß sie für die Bekämpfung von DIABROTICA und den anderen Bodenparasiten vorgesehen sind.

12. Akarizide und nematizide Zusammensetzungen, dadurch gekennzeichnet, daß sie als Wirkstoff zumindest eine Verbindung, wie in einem der Ansprüche 1 bis 7 definiert, enthalten.

13. Assoziationen mit insektizider, akarizider oder nematizider Aktivität, dadurch gekennzeichnet, daß sie als Wirkstoff einesteils zumindest eine Verbindung der allgemeinen Formel (I) und andererseits zumindest einen Ester der Pyrethrinoide, ausgewählt unter den Estern des Allethrolons, des 3,4,5,6-Tetrahydrophthalimidomethylalkohols, des 5-Benzyl-3-furylmethylalkohols, des 3-Phenoxybenzylalkohols und des α-Cyano-3-phenoxybenzylalkohols der Chrysanthemumsäuren, unter den Estern des 5-Benzyl-3-furylmethylalkohols der 2,2-Dimethyl-3-(2-oxo-3-tetrahydrothiophenylidenmethyl)-cyclopropancarbonsäuren, unter den Esfern des 3-Phenoybenzylalkohols und des α-Cyano-3-phenoxybenzylalkohols der 2,2-Dimethyl-3-(2,2-dichlorvinyl)-cyclopropancarbonsäuren, unter den Estern des α-Cyano-3-phenoxybenzylalkohols der 2,2-Dimethyl-3-(2,2-dibromvinyl)-cyclopropancarbonsäuren, unter den Estern des 3-Phenoxybenzylalkohols der 2-Parachlorphenyl-2-isopropylessigsäuren, unter den Estern des Allethrolons, des 3,4,5,6-Tetrahydrophthalimidomethylalkohols, des 5-Benzyl-3-furylmethylalkohols, des 3-Phenoxybenzylalkohols und des α-Cyano-3-phenoxybenzylalkohols der 2,2-Dimethyl-3-(1,2,2,2-tetrahaloethyl)-cyclopropancarbonsäuren, enthalten, worin "halo" für ein Fluor-, Chlor- oder Bromatom steht, mit der Maßgabe, daß die Verbindungen der Formel (I) in sämtlichen ihrer möglichen stereoisomeren Formen ebenso wie die Säure- und Alkoholverknüpfungen der vorstehenden Pyrethrinoidester vorliegen können.
